# FASCICULE DE BREVET EUROPEEN

(11) **EP 0 350 407 B1**
(45) Date de publication et mention de la délivrance du brevet: **22.09.1993**
(21) Numéro de dépôt: 89401959.5
(22) Date de dépôt: 07.07.1989
(51) Int. Cl.: G01N 33/545, G01N 33/547, G01N 33/546, G01N 33/74, C12N 11/08, C12Q 1/68, G01N 33/569

(54) **Réactif sous forme de support solide permettant de fixer par covalence un ligand biologique aminé, sa préparation et son utilisation**
Ein Festphasen-Reagens zur kovalenten Fixierung eines aminierten biologischen Liganden, dessen Herstellung und Verwendung
Solid-phase reagent for the covalent fixation of an aminated biological ligand, its preparation and use

(30) Priorité: 08.07.1988 FR 8809335
(43) Date de publication de la demande: 10.01.1990
(73) Titulaire: BIO MERIEUX Société anonyme dite:, F-69260 Charbonnieres les Bains (FR)
(72) Inventeur: Theretz, Alain, F-69130 Ecully (FR); Aubry, Claude, F-69890 La Tour de Salvagny (FR); Guyot, Alain, F-69006 Lyon (FR); Pichot, Christian, F-69960 Corbas (FR); Le Perchec, Pierre, F-69003 Lyon (FR)
(74) Mandataire: Tonnellier, Jean-Claude

(56) Documents cités:
- EP-A- 0 134 660
- EP-A- 0 161 464
- EP-A- 0 187 391
- EP-A- 0 263 184
- DE-A- 2 255 622

## Description

La présente invention a pour objet un réactif sous forme de support solide sur lequel sont fixées des chaînes latérales hydrophiles réactives, sa préparation et son application, notamment comme réactif biologique.

L'invention a en particulier pour objet un réactif sous forme de support solide permettant de fixer par covalence un ligand biologique aminé, ainsi que le réactif obtenu après fixation dudit ligand biologique.

Il est connu de fixer des ligands biologiques sur des supports solides, notamment pour obtenir des réactifs permettant de révéler la présence ou de doser des molécules en solution ayant une affinité pour ledit ligand.

Par exemple, la fixation d'anticorps ou d'antigènes à la surface de particules de latex est un moyen simple et rapide de visualiser la formation du complexe antigène-anticorps en amplifiant sa taille. Généralement, les molécules d'anticorps ou d'antigène sont adsorbées physiquement sur les particules par liaisons hydrophobes. On ne peut cependant pas éviter une désorption au moins partielle au cours du temps. Il en résulte une variation de la sensibilité du réactif.

La fixation de ligands par liaison covalente sur des supports solides portant en surface des fonctions réactives permet de remédier partiellement à cette difficulté. Il faut remarquer toutefois que le phénomène d'adsorption du ligand subsiste. En outre, cette méthode présente certains inconvénients. Le problème de l'accessibilité des sites actifs du ligand fixé n'est pas résolu car il dépend de la position aléatoire du site de fixation du ligand. Il en résulte que les réactifs obtenus ne sont pas homogènes de ce point de vue. En outre, la fixation du ligand nécessite généralement l'intervention d'agents activateurs ou d'agents de couplage tels que les dialdéhydes, le bromure de cyanogène, les carbodiimides, la benzoquinone, etc..., afin de créer une fonction réactive nécessaire au couplage par covalence du ligand.

D'autres difficultés observées à propos de la fixation de ligands biologiques sur des supports de polymères hydrophobes tels que le polystyrène sont évoquées notamment dans l'article de R.COUTURIER et al., Makromol. Chem. 187, 1603-1610 (1986).

La présente invention permet de remédier à ces inconvénients en fournissant un réactif sous forme de support solide dans lequel la fonction réactive, qui réagit avec le ligand sans utilisation d'un agent de couplage ou d'un activateur, est fixée à l'extrémité d'un bras espaceur hydrophile. La fonction réactive se trouve ainsi éloignée du support solide proprement dit, et cet éloignement, lié à la souplesse du bras espaceur, permet une meilleure accessibilité des sites réactifs du ligand après fixation de celui-ci par covalence. En outre, la présence des bras hydrophiles permet d'éviter ou de réduire fortement le phénomène d'adsorption du ligand et/ou de la molécule à détecter sur le support hydrophobe. Le bras espaceur a de préférence une structure moléculaire linéaire.

La présente invention a donc pour objet un réactif, caractérisé par le fait qu'il est constitué essentiellement par un support solide, en polymère hydrophobe, à la surface duquel sont fixées par covalence des chaînes latérales hydrophiles de formule I 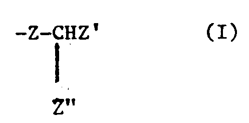
dans laquelle Z est un bras espaceur organique hydrophile non hydroxylé dont la chaîne comporte de 4 à 70 atomes de carbone, Z′ et Z˝ représentent ensemble =O, un groupement protecteur du groupement carbonyle, ou un groupement =YL dans lequel Y représente =N- ou = 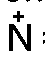 = et L représente le reste d'un ligand aminé de formule schématique L-NH₂ ou L=NH, ou bien Z˝ représente H et Z′ représente -NH-L, L étant le reste dudit ligand.

Dans des modes de réalisation particuliers, le réactif de l'invention peut encore présenter les caractéristiques suivantes, prises isolément ou en combinaison :
- le groupement Z est un groupement polyoxyéthyléné comportant au moins deux motifs oxyéthylène, et en particulier de 2 à 30 motifs ;
- ledit polymère hydrophobe est le polystyrène, le poly-vinyltoluène, le polypropylène, le chlorure de polyvinyle, le chlorure de polyvinylidène, ou leurs copolymères ;
- le groupement protecteur du groupement carbonyle est par exemple un groupement acétal, ou un groupement oxazine ou oxazoline ; dans le cas d'un acétal, Z′ et Z˝ peuvent représenter chacun un groupement alcoxy inférieur (notamment méthoxy ou éthoxy) ou représenter ensemble un groupement éthylènedioxy.

Lorsque le réactif de l'invention contient des chaînes latérales de formule I à terminaison = YL ou -NH-L, il contient également des chaînes latérales de formule I, pour lesquelles Z′ et Z˝ représentent=O, et/ou des chaînes latérales de formule II 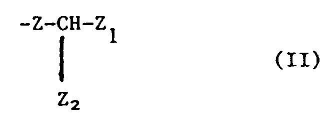
dans laquelle Z est défini comme précédemment, Z₁ représente H et Z₂ représente -OH ou -NHR, ou bien Z₁ et Z₂ représentent ensemble le groupement =N-R, R étant un alkyle inférieur, éventuellement hydroxylé, ayant 1 à 4 atomes de carbone. Le substituant R est de préférence tel que l'amine RNH₂ est la méthylamine, l'éthylamine, l'éthanolamine ou le 2-amino 1-propanol.

La raison de la présence éventuelle de chaînes latérales de formule II, dans le réactif de l'invention, sera exposée ci-après à l'occasion de la description du procédé d'obtention de ce réactif.

Lorsque le réactif de l'invention ne contient pas de chaînes latérales de formule I à terminaison =YL ou -NH-L, il contient des chaînes latérales de formule I avec Z′ et Z˝ représentant =O ou un groupement protecteur du carbonyle, tel que défini précédemment, et éventuellement des chaînes latérales de formule II avec Z₁=H et Z₂=OH, étant entendu que lesdites chaînes de formule I représentent au moins 25% de la totalité des chaînes latérales présentes sur le réactif. Les réactifs pour lesquels Z′ et Z˝ représentent un groupement protecteur sont des précurseurs du réactif aldéhydique correspondant.

Dans le réactif de l'invention, la densité de la totalité des chaînes latérales hydrophiles de formules I et II, rapportée à la surface du réactif, peut varier par exemple de 10⁻⁵ à 10⁻¹⁰ mole de chaînes latérales I et II par m² environ, et en particulier de 10⁻⁵ à 10⁻⁷ mole/m².

Le réactif de l'invention se présente notamment sous forme de billes, et en particulier sous la forme de microbilles dont les dimensions peuvent aller de 0,2 à 1 micromètre environ et notamment de 0,25 à 0,8 micromètre. De préférence, ces microbilles sont calibrées et présentent un indice de polydispersité faible, par exemple inférieur à 1,005.

L'invention a également pour objet un procédé de préparation d'un réactif tel que défini précédemment. Ce procédé est caractérisé par le fait que l'on prépare ledit support solide par polymérisatîon ou copolymérisation d'un ou plusieurs monomères insaturés hydrophobes, selon les méthodes de polymérisation usuelles, et qu'avant la fin de la polymérisation, on ajoute au milieu réactionnel un monomère à chaîne hydrophile dont une extrémité comporte une double liaison copolymérisable avec lesdits monomères insaturés hydrophobes et dont l'autre extrémité comporte un groupement terminal de formule -Z-CHZ₃(Z₄), dans laquelle Z (constituant la chaîne hydrophile) est défini comme ci-dessus, et Z₃ et Z₄ représentent =O ou un groupement protecteur du carbonyle, tel que défini précédemment, ledit monomère hydrophile étant éventuellement en mélange avec un monomère hydrophile analogue à groupement terminal -Z-CH₂OH, que, dans le cas où Z₃ et Z₄ représentent un groupement protecteur du carbonyle, on effectue une réaction de déprotection, que, si désiré, on fait réagir le réactif obtenu, qui comporte des chaînes latérales de formule III :

-Z-CH=O (III)

avec un ligand aminé, selon les méthodes connues, pour obtenir un réactif correspondant dans lequel une partie des chaînes latérales répondent à la formule IV :

-Z-CHY'L (IV)

dans laquelle Z est défini comme précédemment, Y′ représente un groupement =N- lié par sa double liaison au groupement CH, ou Y′ représente un groupement = 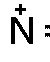 =, et L représente le reste de la molécule de ligand,
que, si désiré, on fait réagir le réactif obtenu avec une amine primaire de formule RNH₂, R étant un alkyle inférieur éventuellement hydroxylé ayant 1 à 4 atomes de carbone, de façon à transformer les chaînes latérales -Z-CH=O, qui n'ont pas réagi avec le ligand, en chaînes correspondantes de formule V :

-Z-CH=N-R (V),

et que, dans le cas où le réactif comporte des chaînes latérales de formule IV et éventuellement de formule V, on soumet, si désiré, le réactif obtenu à l'action d'un agent réducteur capable de réduire les groupements imine ou imminium, pour obtenir un réactif correspondant comportant des chaînes latérales de formule VI :

-Z-CH₂-NH-L (VI)

et le cas échéant des chaînes latérales de formule VII:

- Z - CH₂-NH-R (VII).

On voit que dans la formule IV, L est le reste d'un ligand qui peut être représenté par la formule L-NH₂ (lorsque le ligand a réagi avec l'aldéhyde par un groupement amine primaire), ou L est le reste d'un ligand qui peut être représenté par la formule L=NH, dans laquelle =NH est l'imine d'un groupement de type guanidine contenu dans le ligand (lorsque le ligand a réagi avec l'aldéhyde par ledit groupement de type guanidine).

Dans le cas de la réaction de l'aldéhyde sur un groupement guanidine du ligand, le cation = 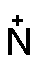 = est accompagné de l'anion OH⁻ formé, qui est toutefois échangeable par tout autre anion désiré, par exemple un anion halogénure.

Dans le cas où l'on ne fait pas réagir le réactif comportant des chaînes latérales de formule V avec l'amine RNH₂, et où l'on soumet le réactif à l'action d'un agent réducteur, les chaînes latérales de formule III présentes sur le réactif peuvent alors être simultanément réduites en chaînes latérales de formule VIII :

-Z-CH₂-OH (VIII).

Dans le procédé décrit ci-dessus, le monomère à chaîne hydrophile peut être ajouté sous la forme d'un mélange avec le monomère hydrophobe.

Le monomère à chaîne hydrophile est par exemple un composé de formule IX : 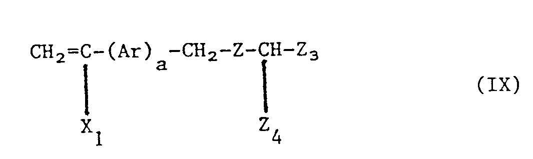
dans laquelle Z, Z₃ et Z₄ sont définis comme précédemment, X₁ représente H ou CH₃, Ar est un groupement m- ou p- phénylène éventuellement substitué par un groupement méthyle, et a=0 ou 1.

Dans le cas où Z est un groupement polyoxyéthyléné, Z est lié au groupement -CH₂- par une liaison éther.

Le monomère IX fournit des réactifs correspondants dans lesquels la chaîne latérale de formule I est rattachée au polymère hydrophobe par l'intermédiaire d'un groupement

-CH₂-CHX₁-(Ar)ₐ-CH₂-.

Le monomère de formule IX peut être préparé par exemple par réaction d'un composé de formule X : 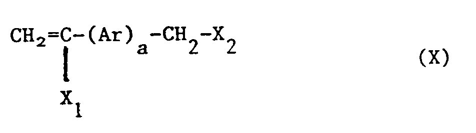
où X₂ est un halogène (notamment chlore ou brome), et X₁ est défini comme ci-dessus, avec un composé de formule XI:

X₄-Z-X₃ (XI)

où Z est défini comme ci-dessus, X₃ représente un groupement -CH=O ou un précurseur de celui-ci, et X₄ est un groupement réactif capable de réagir avec X₂ pour former une liaison entre X et Z.

Lorsque Z est un groupement polyoxéthyléné, X₄ est l'hydrogène de l'hydroxyle terminal du produit XI, qui par réaction avec l'halogène représenté par X₂du produit (X), va former une liaison éther entre les groupements -CH₂- et Z.

Le précurseur du groupement -CH=O est notamment un groupement -CH₂OH qui peut être oxydé en aldéhyde correspondant selon les méthodes usuelles, pour donner un composé de formule IX (avec Z₃ et Z₄ représentant ensemble = O).

Par exemple, lorque X₄-Z-X₃ est le composé

H-(OCH₂CH₂)ₙ₋₁OCH₂-CH₂OH (XI A),

n étant un nombre au moins égal à 2, pouvant varier de 2 à 30 par exemple, la réaction avec le composé X fournit un composé de formule XII : 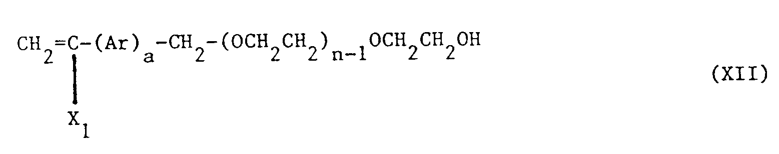
qui par oxydation donne le composé correspondant IX A:

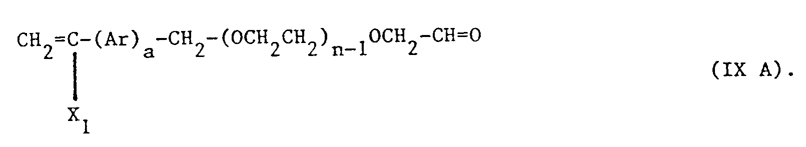

On peut également préparer un composé de formule IX au départ d'un composé XII (sous forme d' alcoolate) par réaction avec un dérivé de formule XIII :

X₂-(CH₂)_{b}-CHZ₃(Z₄) (XIII)

dans laquelle X₂ est un halogène, b=1 ou 2 et Z₃ et Z₄ sont définis comme précédemment, pour obtenir un composé correspondant de formule IX B : 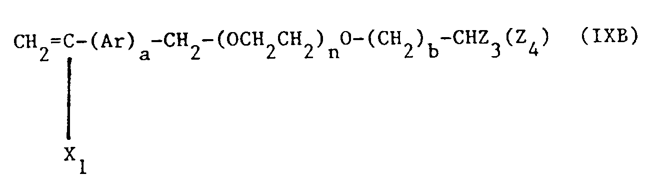
En comparant les formules IX, IX A et IX B, on voit que les composés de formule IX, et en conséquence les composés de formules I à VIII, sont notamment ceux pour lesquels Z représente

- (OCH₂CH₂)ₙ₋₁OCH₂-

ou

- (OCH₂CH₂)ₙO-(CH₂)_{b}-.

Les composés X, XI et XIII sont des composés connus ou pouvant être facilement préparés selon les méthodes connues.

La réaction de polymérisation du monomère hydrophobe et de copolymération du monomère hydrophobe et du monomère à chaîne hydrophile est effectuée selon les méthodes usuelles. En particulier, dans le cas de la préparation d'un réactif sous forme de microbilles, on opère selon la technique connue de polymérisation en émulsion.

La quantité de monomère à chaîne hydrophile ajoutée pendant la réaction de polymérisation est une quantité suffisante pour obtenir la densité souhaitée de chaînes hydrophiles à la surface du réactif. Cette quantité peut être déterminée dans chaque cas par de simples expériences de routine.

A la fin de la réaction de polymérisation, on obtient un réactif portant à sa surface des chaînes latérales de formule III. Il s'agit d'un réactif intermédiaire, qui permet de fixer sur le support hydrophobe du réactif, divers ligands aminés par réaction du groupement aldéhyde sur l'amine du ligand. Pour cela, on met en contact ledit réactif avec une solution d'un ligand aminé comportant des groupements amine primaire et/ou des groupements de type guanidine, pour obtenir un réactif modifié comportant des chaînes latérales de formule IV :

-Z-CH Y'L (IV)

dans laquelle
L est le reste de la molécule du ligand,
Y′ est un groupement =N- lié par sa double liaison au groupement CH lorsque l'aldéhyde a réagi avec un groupement amine primaire du ligand,
et Y′ représente un groupement = 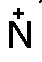 = lorsque l'àldéhyde a réagi avec l'imine d'un groupement de type guanidine du ligand.

Si désiré, on soumet en outre ledit réactif modifié à l'action d'un agent réducteur, tel qu'un borohydrure ou un cyanoborohydrure, capable de réduire les groupements =N- ou = 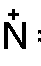 = en groupements amine correspondants.

Les ligands biologiques aminés qui peuvent être fixés sur les réactifs de l'invention selon la méthode décrite ci-dessus sont notamment des protéines, par exemple des anticorps (notamment IgG, IgM, IgE), y compris des anti-anticorps, des antigènes, des haptènes, des hormones peptidiques, des enzymes, ou encore des oligonucléotides, des lectines, des neurotransmetteurs ou des drogues synthétiques ou naturelles analogues à ces derniers (agonistes ou antagonistes).

La quantité de ligand fixée sur le réactif dépend évidemment de l'encombrement du ligand. On cherche généralement à fixer le maximum de ligand sur le réactif. La quantité de ligand à employer est déterminée facilement dans chaque cas par des expériences de routine.

La réaction avec l'agent réducteur n'est pas obligatoire, car le réactif portant les chaînes latérales de formule IV peut être utilisé tel quel, par exemple comme réactif pour détecter la présence du récepteur ou d'un partenaire du ligand.

Bien que cela ne soit généralement pas nécessaire, on peut neutraliser (afin d'éviter toute réaction parasite de fixation du récepteur ou d'un partenaire du ligand, lors de l'utilisation ultérieure du réactif) les groupements aldéhyde restants, sur les chaînes latérales qui n'ont pas réagi avec le ligand, en les faisant réagir avec une amine primaire de faible poids moléculaire RNH₂ (de préférence une alcanolamine), de façon à transformer les chaînes latérales de formule III en chaînes de formule V qui peuvent être réduites, si désiré, en chaînes de formule VII. L'agent réducteur utilisé est le même que pour la réduction des chaînes de formule IV en chaînes de formule VI, et ces réductions peuvent évidemment être effectuées simultanément.

Pour neutraliser les groupements aldéhyde restants, on peut également les réduire en groupements alcool primaire correspondants en utilisant une quantité suffisante d' agent réducteur lors du stade de réduction des bases de Schiff.

Les réactifs de l'invention sur lesquels sont fixés un ligand peuvent être utilisés comme réactifs biologiques ou biochimiques, selon les méthodes connues en soi, par exemple pour rechercher la présence éventuelle du récepteur ou d'un partenaire du ligand dans une solution ou dans un fluide biologique à examiner.

Par exemple lorsque le ligand est un anticorps, notamment un anticorps monoclonal, les réactifs de l'invention permettent de détecter la présence de l'antigène correspondant selon les méthodes connues de sérodiagnostic. Parmi les anticorps susceptibles d'être fixés sur le réactif, on citera en particulier les anticorps spécifiques reconnaissant des antigènes correspondant à des états pathogènes, et notamment les anticorps anti-PDF, anti-méningocoque A, anti-méningocoque B, etc...

Lorsque le réactif est sous forme de microbilles, la présence du partenaire du ligand se traduit par une agglutination. Dans les autres cas, on révèle la présence du partenaire à l'aide de réactifs marqués, par exemple fluorescents ou radioactifs, selon les méthodes usuelles.

On va maintenant décrire plus en détail la réalisation de réactifs selon l'invention, en faisant référence plus spécialement à l'obtention de réactifs de polystyrène portant des bras espaceurs hydrophiles du type polyoxyalkylène les réactifs étant en particulier sous forme de microbilles. Les spécialistes comprendront toutefois que le réactif de l'invention peut également être préparé sous la forme de billes, de bandelettes ou de disques, y compris sous la forme de disques constituant des plaques de microtitration, et que d'autres matériaux hydrophobes, ainsi que d'autres bras hydrophiles, peuvent être utilisés.

Pour réaliser ces réactifs, il convient d'abord de préparer le monomère à chaîne hydrophile. Pour cela, on peut partir d'un dérivé du styrène capable de réagir avec les alcools primaires, par exemple un halogénométhylstyrène tel que le chlorométhylstyrène ou le bromométhylstyrène (isomère méta ou para, ou mélange d'isomères méta et para).Ces dérivés du styrène peuvent réagir avec un polyalkylèneglycol en milieu basique pour former un monomère à chaîne hydrophile selon la réaction suivante :

CH₂=CH-C₆H₄-CH₂Cl+H-(OAlk)ₙ-OH-------> CH₂=CH-C₆H₄-CH₂-(OAlk)ₙOH + HCl Composé (1)

On voit que le composé (1) est un cas particulier des composés de formule XII.

En réalité, dans le milieu basique (hydroxyde de sodium ou de potassium) il ne se forme pas d'acide chlorhydrique mais le sel alcalin correspondant.

Dans la réaction ci-dessus, Alk représente un groupement éthylène, et n est un nombre au moins égal à 2, pouvant varier par exemple de 2 à 30.

Le composé (1) formé est copolymérisable avec le styrène.

La réaction d'oxydation de l'alcool primaire terminal du composé (1) est effectuée selon les méthodes connues. On utilise en particulier une méthode d'oxydation mettant en oeuvre le diméthylsulfoxyde (DMSO) activé. Cette technique, très employée pour l'oxydation des alcools en dérivés carbonylés, implique l'attaque du DMSO par un dérivé électrophile pour donner un complexe DMSO "activé" :

(CH₃)₂S+-O⁻+ E ----> (CH₃)₂-S⁺-O-E,

E représente le dérivé électrophile.

L'attaque par un nucléophile, dans le cas présent un alcool primaire du type R′CH₂OH, R′ étant le reste de la molécule d'alcool primaire, est alors facilitée :

(CH₃)₂S⁺-O-E + HO-CH₂-R' ----> (CH₃)₂S⁺-O-CH₂-R'+OE⁻

L'action d'une base sur l'alkoxysulfoxonium formé donne le compose carbonylé selon la réaction :

(CH₃)₂S⁺-O-CH₂-R'+ base ----> CH₃-S-CH₃ + O = CH-R'.

Les agents électrophiles qui activent le DMSO sont relativement nombreux. On peut citer par exemple l'anhydride trifluoroacétique, l'anhydride acétique, le chlorure de benzoyle, le chlorure d'oxalyle, les carbodiimides tels que la dicyclohexylcarbodiimide (DCC), etc...

La base utilisée est par exemple une amine tertiaire telle que la triétylamine.

Les réactions d'oxydation des alcools primaires avec le DMSO activé sont décrites par exemple dans les documents suivants A.J. MANCUSO et al., Synthesis, 1981, pp.165-185, et K.E. PFITZNER et al., J.Am.Chem.Soc., 87 (24), 1965, pp.5662-5678.

La réaction d'oxydation permet donc d'obtenir le monomère ayant la formule suivante (2) :

CH₂=CH-C₆H₄-CH₂-(OAlk)ₙ₋₁OCH₂-CH=O (2).

Lorsque la réaction d'oxydation n'est pas totale, le produit (2) est obtenu en mélange avec le produit (1) de départ. De tels mélanges peuvent être utilisés pour la réaction de copolymérisation ultérieure. De préférence, ces mélanges doivent comporter au moins 25% de produit (2).

La copolymérisation du produit (2) avec le polystyrène conduit à l'obtention d'un copolymère dont les motifs dérivés de (2) comportent, en position méta ou para, un substituant -CH₂-Z-CH=O, Z étant défini comme précédemment. Autrement dit, le réactif obtenu est alors constitué d'un support solide en polystyrène à la surface duquel sont fixées des chaînes latérales -CH₂-Z-CR=O.

Lorsqu'on utilise pour la copolymérisation des mélanges de produits (1) et (2), le réactif obtenu est un réactif dont une partie des chaînes latérales auront une terminaison -CH₂OH au lieu de -CH=O.

Pour réaliser des microbilles à la surface desquelles sera greffé le bras porté par le monomère (2), on effectue une polymérisation en émulsion avec le monomère de base, par exemple le styrène. Puis, avant que la réaction de polymérisation soit terminée, par exemple lorsque 75 à 95% (de préférence 85 à 95%) du monomère de base sont polymérisés, on ajoute le monomère (2) en quantité suffisante pour obtenir par copolymérisation avec le styrène restant, des microbilles à la surface desquelles sont greffés des bras hydrophiles du type :

-(OAlk)ₙ₋₁-OCH₂-CH=O .

De préférence, le monomère (2) est ajouté sous la forme d'un mélange avec le monomère de base, tel que le styrène.

Le principe des réactions de polymérisation en émulsion est connu en soi. Ce procédé consiste à réaliser une émulsion aqueuse du monomère à l'aide d'un agent tensioactif, et à effectuer la polymérisation par addition d'un générateur de radicaux libres soluble en phase aqueuse. Il se forme une dispersion de particules submicroniques de polymère, appelées latex. Le générateur de radicaux libres peut être un persulfate, par exemple K₂S₂O₈, ou un dérivé azo hydrosoluble tel que l'acide 4,4′-azobis 4-cyanopentanoïque.

Pour obtenir des particules de latex ayant une bonne homogénéité de dimensions, il convient d'opérer en présence d'une quantité contrôlée d'agent tensioactif. La concentration d'agent tensioactif à utiliser peut être déterminée dans chaque cas par des expériences de routine.

Les conditions de polymérisation en émulsion sont décrites par exemple par D. C. BLACKLEY dans "Emulsion Polymerisation", Applied Science Publishers, (1975), et par J.W. VANDERHOFF et al, J. Macromol. Sci.-Chem., A7 (3), pp.667-670 (1973).

Parmi les agents tensioactifs utilisables, on peut citer par exemple les tensioactifs anioniques ou, de préférence, zwitterioniques, en particulier des bêtaïnes telles que les sulfobêtaïnes décrites dans le brevet US n°4.704.229, et en particulier l'acétyl-4 aza-4 (N,N-diméthyloctadécyl ammonium)-6 hexane sulfonate.

Pour fixer un ligand biologique aminé sur les réactifs obtenus selon l'invention, qui comportent, après déprotection éventuelle, des chaînes latérales à terminaison aldéhyde, on met en contact le réactif avec une solution du ligand. Le groupement aldéhyde agit sur les groupements amines primaires du ligand pour former une base de Schiff que l'on réduit, si désiré, en amine secondaire à l'aide d'un agent réducteur tel qu'un borohydrure ou cyanoborohydrure de métal alcalin ou d'ammonium. Si le ligand comporte d'autres fonctions azotées telles que la fonction guanidine (ou son sel de guanidinium), la condensation avec l'aldéhyde donnera une base de Schiff comportant une fonction iminium susceptible d'être réduite en amine secondaire à l'aide des mêmes agents réducteurs ; voir par exemple KING, Biochemistry, Vol.5,3454 (1966), et NAKAYA et al., J.BIOCHEM. Tokyo, 61, 345 (1967).

Pour faire réagir le réactif avec le ligand, on opère généralement en solution aqueuse à une température pouvant aller par exemple de 4 à 60°C. La fin de la réaction de couplage avec le ligand est généralement signalée par une stabilisation du signal de lumière diffusée par les particules.

Les réactifs contenant le ligand fixé par covalence peuvent être utilisés, par exemple, selon les méthodes connues, dans le domaine de la biologie ou de la biochimie, en particulier dans le domaine du diagnostic médical fondé sur des tests sérologiques, tels que ceux utilisant la formation de complexes antigène-anticorps. Lorsque le ligand fixé est une enzyme, le réactif peut être employé comme catalyseur dans les réactions biologiques, chimiques ou biochimiques catalysées par cette enzyme.

Les exemples suivants illustrent l'invention sans toutefois la limiter.

### EXAMPLE 1: Préparation du monomère à chaîne hydrophile terminée par une fonction alcool primaire.

### a) Préparation d'un monomère avec chaîne hydrophile de masse molaire moyenne Mn = 600 terminée par une fonction alcool primaire

On sèche 0,29 mole de polyéthylèneglycol ayant une masse molaire moyenne de 600 (PEG 600) par entraînement azéotropique de l'eau par le benzène (point d'ébullition de l'azéotrope = 69°C) ou le toluène (point d'ébullition de l'azéotrope = 85°C).

Dans un tricol, on place 0,275 mole (11g) de NaOH et 0,25 mole (150 g) de PEG 600 séché. On agite jusqu'à complète disparition de la soude. La température est amenée à 40°C et on ajoute goutte à goutte 0,3 mole de chlorométhylstyrène (42,3 ml, mélange d'isomères 40% para et 60% méta). La température est maintenue à 40°C maximum pendant 18 heures. La solution est diluée dans du chloroforme, filtrée pour éliminer le sel formé (NaCl), lavée successivement par une solution d'acide chlorhydrique à 1,5 (500ml), puis à l'eau distillée jusqu'à pH neutre, séchée par du sulfate de sodium anhydre et filtrée. Après évaporation du solvant, le produit est lavé par l'heptane afin d'éliminer le chlorométhylstyrène résiduel. L'heptane est éliminé par évaporation et le produit final (rendement 80%; Mn = 716) est caractérisé par ¹H - RMN et spectroscopie infrarouge. Il peut être stocké à -20°C pendant plusieurs mois.

Dans le mode opératoire ci-dessus, on peut remplacer la soude par de la potasse (0,275 mole, 15,4 g), le chloroforme par le dichlorométhane ou le benzène, et l'heptane par l'hexane ou le pentane.

Le produit final est appelé le VOE 600.

### b) Préparation de monomères analogues à ceux de l'exemple 1a, avec d'autres chaînes hydrophiles de masse moyenne Mn inférieure à 600.

Selon une méthode analogue à celle décrite à l'exemple 1a, on sèche 0,29 mole de PEG dont les masses moyennes sont : 106, 200, 300 et 400 respectivement. Les conditions de couplage et de purification sont semblables à celles de l'exemple 1a.

On obtient les produits finals suivants : VOE 106 (Mn = 222), VOE 200 (Mn = 316), VOE 300 (Mn = 416) et VOE 400 (Mn = 516) que l'on caractérise par ¹H - RMN et spectroscopie infrarouge.

Les VOE 106 et VOE 200 ne sont pas lavés par l'heptane car ils sont solubles dans ce solvant ou les analogues (hexane, pentane). Le chlorométhylstyrène résiduel est éliminé dans ces cas par évaporation à 50°C sous pression réduite (0,5 mm Hg, soit environ 65 Pa).

### c) Préparation de monomères analogues à ceux de l'exemple 1a, avec chaîne hydrophile de masse moyenne Mn supérieure à 600.

A la différence des précédents, les PEG de masse supérieure à 600 (1.000, 1.500...) sont solide à température ambiante.

Le PEG 1.500 est séché comme décrit dans l'exemple 1a.

On place 0,133 mole de PEG 1.500 (200g) dans une ampoule de coulée à double enveloppe thermostatée à 50°C, montée sur un tricol chauffé à 50°C et contenant 0,147 mole d'hydrure de sodium (4,224 g de NaH avec 10% de paraffine) dans 100 ml de tétrahydrofuranne (THF) sec, fraîchement distillé. Le PEG 1.500 est ajouté goutte à goutte, de manière à éviter un trop fort dégagement d' hydrogène. Après 18 heures d'agitation, on ajoute 0,16 mole (22,55 ml) de chlorométhylstyrène et l'agitation est maintenue pendant 24 heures à 50°C.

Le mélange est dilué dans le THF et filtré à chaud. On ajoute de l'héptane au filtrat, jusqu'à fin de précipitation. On filtre. Le précipité est repris dans le THF et reprécipité plusieurs fois. Après évaporation du solvant, le produit est caractérisé comme précédemment (VOE 1.500, Mn = 1.616). On peut remplacer le PEG 1.500 par d'autres PEG de masses supérieures à 600, et obtenir les macromères correspondants selon le même principe.

### EXEMPLE 2 : Préparation de monomères à chaîne hydrophile terminée par une fonction aldéhyde.

### EXEMPLE 2.1 : Oxydation par le complexe DMSO/Anhydride Acétique

### a) Oxydation du VOE 600

On ajoute 7ml d'anhydride acétique à 198 g de diméthylsulfoxyde. On agite pendant 30 minutes à température ambiante.

On ajoute 0,021 mole (15,4 g) du monomère obtenu à l'exemple 1a et on maintient l'agitation pendant 40 heures.

### b) Oxydation selon le même procédé, des monomères obtenus aux exemples 1b et 1c

On obtient de la même manière, les monomères analogues à ceux décrits aux exemples 1b et 1c porteurs d'un groupement aldéhyde terminal.

### EXEMPLE 2.2 : Autre préparation des monomères à chaîne hydrophile terminée par une fonction aldéhyde

### a) Oxydation du VOE 600

A 60mmoles de diméthylsulfoxyde (4,255ml dans 25ml de CH₂Cl₂) préalablement séché sur alumine basique, on ajoute lentement, à -60°C, 55mmoles de chlorure d'oxalyle (4,8ml dans 125ml de CH₂Cl₂). Après 10 minutes d'agitation on ajoute 50mmoles du produit de l'exemple 1a en solution dans 50ml de CH₂Cl₂, de façon à ne pas dépasser la température de -60°C, et on maintient l'agitation pendant 15 minutes. On ajoute alors 250 mmoles (35ml) de triéthylamine. On laisse remonter la température à 20°C et ajoute 150ml d'eau distillée. On sépare la phase aqueuse. La phase organique est lavée successivement par 200ml d'eau distillée, par une solution saturée en chlorure de sodium, par une solution aqueuse d'acide chlorhydrique à 1%, à nouveau par l'eau distillée, par une solution à 5% de K₂CO₃, et enfin à l'eau distillée.

Après séchage et évaporation du solvant, le produit est caractérisé en 1H-RMN et infrarouge.

### b) Oxydation selon le même procédé, des monomères obtenus a l'exemple 1b.

On obtient selon le même procédé, les monomères à groupement terminal aldéhyde analogues des VOE 106, VOE 200, VOE 300 et VOE 400.

### EXEMPLE 2.3 : Autre préparation du monomère de l'exemple 2.1

### a) Oxydation du VOE 600

On ajoute 6,19g (30mmoles) de dicyclohexylcarbodiimide à 15ml de diméthylsulfoxyde (210 mmoles). 0n agite pendant 2 heures, puis ajoute 10 mmoles du produit de l'exemple 1a. Après 5 minutes d'agitation on ajoute 5 mmoles d'acide phosphorique. La solution est agitée pendant 24 heures puis filtrée afin d'éliminer le précipité de dicyclohexylurée formé. Le résidu est repris dans le chlorure de méthylène et lavé plusieurs fois à l'eau. Le solvant est éliminé et le résidu est repris dans l' acétone. On filtre plusieurs fois jusqu'à ne plus obtenir de cristaux de dicyclohexylurée.

La fonction aldéhyde est caractérisée en infrarouge (C=O à 1710 cm⁻¹) et en 1H-RMN (pic à 9,9-10 ppm).

### b) Oxydation selon le même procédé, des monomères obtenus a l'exemple 1b.

On obtient selon le même procédé, les analogues à groupement terminal aldéhyde des monomères VOE 106, VOE 200, VOE 300 et VOE 400.

### c) Oxydation selon le même procédé, des monomères obtenus à l'exemple 1c

On obtient selon le même procédé, les analogues à groupement terminal aldéhyde des monomères VOE 1.000, VOE 1.500 et de masses supérieures, à la différence que le résidu est repris dans le THF, filtré à chaud et précipité plusieurs fois à l'heptane, l'hexane ou le pentane.

### EXAMPLE 3 : Synthèse de particules de latex comportant des chaînes latérales hydrophiles à groupement aldéhyde terminal

### EXEMPLE 3.1: Synthèse de particules de latex comportant des chaînes hydrophiles provenant du produit décrit dans l'exemple 2.1a.

A 826g d'eau distillée dégazée on ajoute 0,1g d'acétyl-4 aza-4 (N,N-diméthyloctadécyl ammonium)-6 hexane sulfonate (tensioactif) et 0,3g d'hydrogénocarbonate de sodium. On agite pendant 45 minutes à 70°C sous courant d'azote puis ajoute 104,15g de styrène. Après agitation à 70°C pendant 60 minutes, on ajoute 0,7g de persulfate de potassium (initiateur) et on maintient l'agitation à 70°C pendant 3 heures. On ajoute alors 25g d'un mélange 50/50, en poids, de styrène et du produit de l'exemple 2. 1a, ainsi qu'une nouvelle quantité (0,2g) d'initiateur. On maintient l'agitation à 70°C pendant 12 heures.

On obtient ainsi des microsphères calibrées d'un copolymère poly (styrène/VOE 600 oxydé) portant des chaînes hydrophiles terminées par une fonction aldéhyde (densité surfacique moyenne de chaînes : 3,3/nm²).

Les particules sont caractérisées par mesure de diffusion de la lumière et par microscopie électronique. Ces particules ont des tailles comprises entre 250 et 350nm, avec un très faible indice de polydispersité (inférieur à 1,005). La présence des chaînes latérales hydrophiles à la surface du latex est mise en évidence par analyse infrarouge et par analyse ESCA. La présence d'aldéhyde est mise en évidence par une coloration rose obtenue par le réactif de Schiff spécifique des aldéhydes.

Le nombre moyen de chaînes latérales hydrophiles par particule peut être déterminé par réaction avec une molécule aminée radioactive (par exemple ¹²⁵I-triiodothyronine, ¹⁴C-éthanolamine, immunoglobuline marquée à l'iode 125).
b) Selon le même procédé, on prépare des particules de latex de polystyrène calibrées portant en surface des chaînes hydrophiles terminées par une fonction aldéhyde, de densité surfacique moyenne : 1,6 chaine/nm², en remplaçant le mélange 50/50 par un mélange 75/25 (en poids) de styrène et du produit de l'exemple 2.1a.
c) Selon le même procédé, on prépare des particules de latex de polystyrène calibrées, portant en surface des chaînes hydrophiles terminées par une fonction aldéhyde, de densité surfacique moyenne = 0,8 chaîne/nm², en utilisant 25g d'un mélange 87,5/12,5 (en poids) de styrène et du produit de l'exemple 2.1a.
d) Selon le même procédé, on prépare des particules de latex de polystyrène, calibrées, portant en surface des chaînes hydrophiles terminées par une fonction aldéhyde, de densité surfacique moyenne= 0,3 chaîne/nm² en utilisant 25g d'un mélange 95/5 (en poids) de styrène et du produit de l'exemple 2.1a.

### EXEMPLE 3.2 : Synthèse de latex comportant des chaînes latérales hydrophiles provenant des produits décrits dans l'exemple 2.1b

a) On obtient selon un procédé analogue à celui décrit dans l'exemple 3.1a des latex correspondants, portant des chaînes hydrophiles à groupement terminal aldéhyde sous la forme de particules calibrées de copolymères poly (styrène/VOE 106), poly(styrène/VOE 200), poly(styrène/VOE 300) et poly(styrène/VOE 400), de densité surfacique moyenne : environ 3,3 chaînes hydrophiles/nm², en ajoutant respectivement, au stade final, 25g d'un des mélanges suivants :

| | Sty (g) | VOE x(g) |
|---|---|---|
| poly (sty / VOE 106) | 21,1 | 3,9 |
| poly (sty / VOE 200) | 19,5 | 5,5 |
| poly (sty / VOE 300) | 17,7 | 7,3 |
| poly (sty / VOE 400) | 16 | 9 |
| Sty est l'abréviation de styrène. | | |

b) On obtient selon un procédé analogue à celui décrit dans l'exemple 3.1b des latex correspondants, portant des chaînes hydrophiles à groupement terminal aldéhyde, sous forme de particules calibrées de copolymères poly(styrène/VOE 106), poly(styrène/VOE 200), poly(styrène/VOE 300) et poly(styrène/VOE 400) de densité surfacique : environ 1,6 chaîne hydrophile/nm², en ajoutant respectivement, au stade final 25 g d'un des mélanges suivants :

| | Sty (g) | VOE x(g) |
|---|---|---|
| poly (sty / VOE 106) | 23 | 2 |
| poly (sty / VOE 200) | 22,2 | 2,8 |
| poly (sty / VOE 300) | 21,4 | 3,6 |
| poly (sty / VOE 400) | 20,5 | 4,5 |

c) On obtient selon un procédé analogue à celui de l'exemple 3.1c des latex correspondants, portant des chaînes hydrophiles à groupement terminal aldéhyde, sous la forme de particules calibrées de copolymères poly(styrène/VOE106)) poly(styrène/VOE 200), poly(styrène/VOE 300) et poly(styrène/VOE 400) de densité surfacique moyenne = 0,8 chaîne hydrophile /nm² environ, en ajoutant respectivement au stade final, 25 g d'un des mélanges suivants :

| | Sty (g) | VOE x(g) |
|---|---|---|
| poly (sty / VOE 106) | 24 | 1 |
| poly (sty / VOE 200) | 23,6 | 1,4 |
| poly (sty / VOE 300) | 23,2 | 1,8 |
| poly (sty / VOE 400) | 22,7 | 2,3 |

On obtient selon un procédé analogue à celui décrit dans l'exemple 3.1d des latex correspondants, portant des chaînes hydrophiles à groupement terminal aldéhyde, sous forme de particules calibrées de copolymères poly(styrène/VOE106), poly(styrène/VOE 200), poly(styrène/VOE 300) et poly(styrène/VOE 400) de densité surfacique moyenne: 0,3 chaîne hydrophile/nm² environ, en ajoutant respectivement au stade final, 25 g d'un des mélanges suivants :

| | Sty (g) | VOE x(g) |
|---|---|---|
| poly (sty / VOE 106) | 24,6 | 0,4 |
| poly (sty / VOE 200) | 24,5 | 0,5 |
| poly (sty / VOE 300) | 24,3 | 0,7 |
| poly (sty / VOE 400) | 24,1 | 0,9 |

### EXEMPLE 4 : Fixation d'anticorps

### Préparation d'un réactif latex-anticorps anti-PDF

L'anticorps anti-produits de dégradation du fibrinogène (anti-PDF) est un anticorps monoclonal de type IgGl produit par BIO MERIEUX S.A.

On opère de la façon suivante :
12 mg d'anticorps sont placés en solution dans 18 ml de tampon Tris 0,1M pH 9,0, NaCl 1,25.10⁻²M. On ajoute sous agitation 600 mg (6ml d'une suspension à 10%) du latex (poly(sty/VOE 200) à groupement terminal aldéhyde préparé à l'exemple 3.2c.

Après 3 heures d'agitation, le latex est amené à 1% en poids par addition de 36 ml de tampon tris 0,1 M pH 8,2 NaCl 1,25.10⁻² M, albumine 1,67%, azide de sodium 0,167%.

Pour caractériser la présence de PDF à l'aide du réactif obtenu, on opère par exemple de la façon suivante : Sur une lame de verre propre et sèche, on dépose 30 µl de sérum à tester et 30µl du réactif. Après mélange des deux gouttes à l'aide d'un bâtonnet en plastique, la lame est agitée par un mouvement de rotation. La présence éventuelle de PDF dans le sérum est révélée par une agglutination visible en moins de deux minutes.

Ce réactif détecte des quantités aussi faibles que 500ng/ml de produits de dégradation de la fibrine.

### EXEMPLE 5 : Préparation d'un réactif latex-anticorps anti-méningocoque A (dit anti-Méningo A)

L' anticorps utilisé est un anticorps monoclonal de type IgG3 produit par BI0 MERIEUX S.A.

### Méthode I :

A 100 mg de latex obtenu à l'exemple 3.2a (poly(styrène/VOE 106)), on ajoute 1mg d'anticorps anti-Méningo A dans un tampon acide acétique/acétate 0,1M, pH 5,5, NaCl 0,1M, albumine bovine 0,2%.

Après 5 heures d'agitation, on ajoute 1ml d'une solution de cyanoborohydrure de sodium 8.10⁻³M dans un tampon Tris 0,1M pH 8,2, NaCl 0.1M.

On agite pendant 18 heures, puis la solution est centrifugée à 10.000 rpm pendant 20 minutes et le culot de centrifugation est repris par un tampon Tris 0,1M, NaCl 0,1M, albumine bovine 0,5%, azide de sodium 0,1%, pH 8,2.

Ce réactif détecte des quantités aussi faibles que 10 nanogrammes d'antigène polysaccharidique de Méningo A.

### Méthode II :

A 50mg de latex de l'exemple 3.2c (poly(styrène/VOE 200)), on ajoute 0,5mg d'anticorps anti-Méningo A dans un tampon carbonate/bicarbonate 0,1M pH 9,0 NaCl 0,1M contenant 0,1% d'albumine bovine. Après 5 heures d'agitation, on ajoute 0,5ml d'une solution de borohydrure de sodium 8.10⁻³M dans un tampon Tris 0,1M, NaCl 0,1M, pH 8,2.

Après agitation pendant 18 heures, la solution est centrifugée à 12.500 rpm pendant 15 minutes et reprise dans un tampon Tris 0,1M NaCl 0,1M, albumine 0,5%, azide de sodium 0,1%, pH 8,2.

Ce réactif permet de détecter des quantités aussi faibles que 10 nanogrammes d'antigène polysaccharidique de Méningo A.

### EXEMPLE 6 : Préparation d'un réactif latex-anticorps anti-Méningocoque B (dit anti-Méningo B)

L'anticorps utilisé est un anticorps monoclonal de type IgM, produit par BIO MERIEUX S.A.

A 20mg de latex obtenu à l'exemple 3.1c (poly(styrène/VOE 600), on ajoute 800 microgrammes d'anticorps en solution dans un tampon acide acétique/acétate 0,1M, pH 5,5, NaCl 5.10⁻²M, albumine bovine 0,3%, azide de sodium 0,1%. On agite pendant 2 heures.

Ce réactif détecte des quantités aussi faibles que 10 nanogrammes d'antigène polysaccharidique de Méningo B.

## Revendications

1. Réactif caractérisé par le fait qu'il est constitué essentiellement par un support solide, en polymère hydrophobe, à la surface duquel sont fixées par covalence des chaînes latérales hydrophiles de formule I 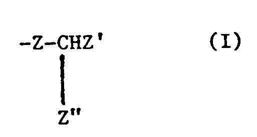 dans laquelle Z est un bras espaceur organique hydrophile non hydroxylé dont la chaîne comporte de 4 à 70 atomes de carbone, Z′ et Z˝ représentent ensemble =O, un groupement protecteur du groupement carbonyle, ou un groupement =YL dans lequel Y représente =N- ou = 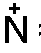 = et L représente le reste d'un ligand aminé de formule schématique L-NH₂ ou L=NH, ou bien Z˝ représente H et Z′ représente -NH-L, L étant le reste dudit ligand.

2. Réactif selon la revendication 1, caractérisé par le fait qu'il contient des chaînes latérales de formule I à terminaison =YL ou -NH-L, et qu'il contient également des chaînes latérales de formule I, pour lesquelles Z′ et Z˝ représentent =O, et/ou des chaînes latérales de formule II 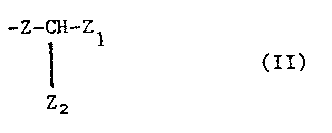 dans laquelle Z est défini comme dans la revendication 1, Z₁ représente H et Z₂ représente -OH ou -NHR, ou bien Z₁ et Z₂ représentent ensemble le groupement =N-R, R étant un alkyle inférieur, éventuellement hydroxylé, ayant 1 à 4 atomes de carbone.

3. Réactif selon la revendication 1 ou 2, caractérisé par le fait qu'il contient des chaînes latérales de formule I, avec Z′ et Z˝ représentant ensemble =O, le reste des chaînes latérales présentes sur le réactif étant des chaînes latérales de formule II, avec Z₁=H et Z₂=OH, étant entendu que lesdites chaînes de formule I représentent au moins 25% de la totalité des chaînes latérales présentes sur le réactif.

4. Réactif selon la revendication 1, caractérisé par le fait que les chaînes latérales de formule I sont telles que Z′ et Z˝ représentent ensemble =O ou un groupement protecteur du groupement carbonyle.

5. Réactif selon la revendication 4, caractérisé par le fait que ledit groupement protecteur est un groupement acétal, oxazine ou oxazoline.

6. Réactif selon l'une quelconque des revendications 1 à 5, caractérisé par le fait que la densité de la totalité des chaînes latérales de formule I et II, rapportée à la surface du réactif, est de 10⁻⁵ à 10⁻¹⁰ mole/m² environ.

7. Réactif selon la revendication 6, caractérisé par le fait que ladite densité est de 10⁻⁵ à 10⁻⁷ mole/m² environ.

8. Réactif selon l'une quelconque des revendications précédentes, caractérisé par le fait que Z est un groupement polyoxyéthyléné comportant au moins 2 motifs oxyéthylène.

9. Réactif selon la revendication 8, caractérisé par le fait que Z représente
-(OCH₂CH₂)ₙ₋₁OCH₂- ou -(OCH₂CH₂)ₙO-(CH₂)_{b}-,
n étant un membre au moins égal à 2 et b étant égal à 1 ou 2.

10. Réactif selon l'une quelconque des revendications précédentes, caractérisé par le fait que ledit polymère hydrophobe est choisi dans le groupe constitué par le polystyrène, le polyvinyl toluène, le polypropylène, le chlorure de polyvinyle, le chlorure de polyvinylidène, et leur copolymères.

11. Réactif selon l'une quelconque des revendications précédentes, caractérisé par le fait que ledit polymère hydrophobe est le polystyrène et que lesdites chaînes latérales sont des chaînes -CH₂-Z-CHZ′ (Z˝) et éventuellement -CH₂-Z-CHZ₁(Z₂), Z, Z′ et Z˝ étant définis comme dans la revendication 1, et Z₁ et Z₂ étant définis comme dans la revendication 2.

12. Réactif selon l'une quelconque des revendications 1, 2 et 6 à 11, caractérisé par le fait qu'il contient des chaînes latérales à terminaison =YL ou -NH-L, Y étant défini comme dans la revendication 1 et L étant le reste d'un ligand aminé choisi parmi les protéines, en particulier les anticorps, les antigènes, les haptènes, les hormones peptidiques, les enzymes ou encore les oligonucléotides, les lectines, les neurotransmetteurs ou les drogues naturelles ou synthétiques agonistes ou antagonistes d'un neurotransmetteur.

13. Réactif selon la revendication 12, caractérisé par le fait que ledit anticorps est un anticorps spécifique reconnaissant des antigènes correspondant à des états pathogènes.

14. Réactif selon la revendication 13, caractérisé par le fait que ledit anticorps est choisi dans le groupe constitué par les anticorps anti-produits de dégradation du fibrinogène, les anticorps anti-méningocoque A et les anticorps anti-méningocoque B.

15. Réactif selon l'une quelconque des revendications précédentes, caractérisé par le fait qu'il se présente sous la forme de billes.

16. Réactif selon la revendication 15, caractérisé par le fait qu'il se présente sous la forme de microbilles ayant des dimensions pouvant aller de 0,2 à 1 micromètre environ.

17. Réactif selon l'une quelconque des revendications précédentes, caractérisé par le fait que les chaînes latérales de formule I sont rattachées au polymère hydrophobe par l'intermédiaire d'un groupement
-CH₂-CHX₁-(Ar)ₐ-CH₂-
où Ar est un groupement m- ou p- phénylène éventuellement substitué par un groupement méthyle, a est un nombre égal à 0 ou 1, et X₁ représente H ou CH₃.

18. Procédé de préparation d'un réactif tel que défini dans l'une quelconque des revendications précédentes, caractérisé par le fait que l'on prépare ledit support solide par polymérisation ou copolymérisation d'un ou plusieurs monomères insaturés hydrophobes, selon les méthodes de polymérisation usuelles, et qu'avant la fin de la polymérisation, on ajoute au milieu réactionnel un monomère à chaîne hydrophile dont une extrémité comporte une double liaison copolymérisable avec lesdits monomères insaturés hydrophobes et dont l'autre extrémité comporte un groupement terminal de formule -Z-CHZ₃(Z₄), dans laquelle Z (constituant la chaîne hydrophile) est défini comme dans la revendication 1, et Z₃ et Z₄ représentent =O ou un groupement protecteur du carbonyle, ledit monomère hydrophile étant éventuellement en mélange avec un monomère hydrophile analogue à groupement terminal -Z-CH₂OH, que, dans le cas où Z₃ et Z₄ représentent un groupement protecteur du carbonyle, on effectue une réaction de déprotection, que, si désiré, on fait réagir le réactif obtenu, qui comporte des chaînes latérales de formule III :
-Z-CH=O (III)
avec un ligand aminé, selon les méthodes connues, pour obtenir un réactif correspondant dans lequel une partie des chaînes latérales répondent à la formule IV :
-Z-CHY'L (IV)
dans laquelle Z est défini comme précédemment, Y′ représente un groupement =N- lié par sa double liaison au groupement CH, ou Y′ représente un groupement = 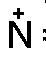 =, et L représente le reste de la molécule de ligand,
que, si désiré, on fait réagir le réactif obtenu avec une amine primaire de formule RNH₂, R étant un alkyle inférieur éventuellement hydroxylé ayant 1 à 4 atomes de carbone, de façon à transformer les chaînes latérales -Z-CH=O, qui n'ont pas réagi avec le ligand, en chaînes correspondantes de formule V :
-Z-CH=N-R (V),
et que, dans le cas où le réactif comporte des chaînes latérales de formule IV et éventuellement de formule V, on soumet, si désiré, le réactif obtenu à l'action d'un agent réducteur capable de réduire les groupements imine ou imminium, pour obtenir un réactif correspondant comportant des chaînes latérales de formule VI :
-Z-CH₂-NH-L (VI)
et le cas échéant des chaînes latérales de formule VII :
- Z - CH₂-NH-R (VII).

19. Procédé selon la revendication 18, caractérisé par le fait que, pour préparer un réactif sous forme de microbilles, on effectue la polymérisation selon la technique de polymérisation en émulsion.

20. Procédé selon la revendication 18 ou 19, caractérisé par le fait que l'on utilise un monomère hydrophile de formule IX : 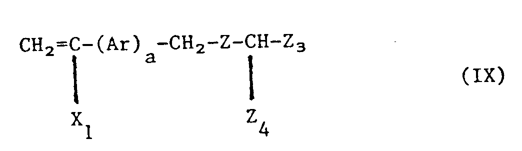 dans laquelle Z, Z₃ et Z₄ sont définis comme dans la revendication 18 et Ar, X₁ et a sont définis comme dans la revendication 17.

21. Utilisation d'un réactif selon l'une quelconque des revendications 1 à 17, caractérisée par le fait que :
a) dans le cas où le réactif ne comporte pas de chaînes latérales de formule I à terminaison =YL ou -NH-L, on met en contact le réactif, comportant des chaînes latérales à terminaison aldéhyde, avec une solution d'un ligand aminé pour obtenir un réactif comportant des chaînes latérales de formule IV :
-Z-CHY'L (IV)
dans laquelle Z et L sont définis comme dans la revendication 1, et Y' est défini comme dans la revendication 18, et, si désiré, on neutralise les groupements aldéhyde restants, sur les chaînes latérales qui n'ont pas réagi avec le ligand, par réaction avec une amine primaire R-NH₂, R étant défini comme dans la revendication 2, et que, si désiré, on soumet le réactif obtenu à l'action d'un agent réducteur capable de réduire les groupements imine ou iminium en groupements amine correspondant ;
et/ou b) à l'aide du réactif contenant des chaînes latérales à terminaison =YL ou -NH-L, L, Y et L étant définis comme dans la revendication 1, on recherche, selon les méthodes connues en soi, la présence éventuelle du récepteur ou d'un partenaire dudit ligand dans une solution ou dans un fluide biologique à examiner ;
ou c) lorsque le ligand fixé sur le réactif est une enzyme, on utilise ledit réactif comme catalyseur dans les réactions biologiques, chimiques ou biochimiques catalysées par ladite enzyme.

## Claims

1. Reagent characterised in that it consists essentially of a solid support, made of hydrophobic polymer, to the surface of which are attached by covalency hydrophilic side chains of formula I 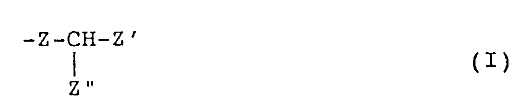 in which Z is a nonhydroxylated hydrophilic organic spacer arm whose chain contains from 4 to 70 carbon atoms, Z' and Z'' together denote =O, a protective group for the carbonyl group, or a =YL group in which Y denotes =N- or = 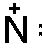 = and L denotes the residue of an amino ligand of schematic formula L-NH₂ or L=NH, or else Z'' denotes H and Z' denotes -NH-L, L being the residue of the said ligand.

2. Reagent according to Claim 1, characterised in that it contains side chains of formula I with a =YL or -NH-L end, and that it also contains side chains of formula I, in the case of which Z' and Z'' denote =O, and/or side chains of formula II 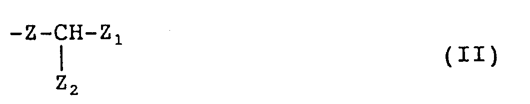 in which Z is defined as in Claim 1, Z₁ denotes H and Z₂ denotes -OH or -NHR, or else Z₁ and Z₂ together denote the =N-R group, R being an optionally hydroxylated lower alkyl containing 1 to 4 carbon atoms.

3. Reagent according to Claim 1 or 2, characterised in that it contains side chains of formula I, with Z' and Z'' together denoting =O, the remainder of the side chains present on the reagent being side chains of formula II, with Z₁ = H and Z₂ = OH, it being understood that the said chains of formula I denote at least 25 % of all the side chains present on the reagent.

4. Reagent according to Claim 1, characterised in that the side chains of formula I are such that Z' and Z'' together denote =O or a protective group for the carbonyl group.

5. Reagent according to Claim 4, characterised in that the said protective group is an acetal, oxazine or oxazoline group.

6. Reagent according to any one of Claims 1 to 5, characterised in that the density of all the side chains of formula I and II, referred to the surface area of the reagent is approximately from 10⁻⁵ to 10⁻¹⁰ mole/m².

7. Reagent according to Claim 6, characterised in that the said density is approximately from 10⁻⁵ to 10⁻⁷ mole/m².

8. Reagent according to any one of the preceding claims, characterised in that Z is a polyoxyethylenated group containing at least two oxyethylene units.

9. Reagent according to Claim 8, characterised in that Z denotes
-(OCH₂CH₂)ₙ₋₁OCH₂- or -(OCH₂CH₂)ₙO-(CH₂)_{b}-,
n being a member equal to at least 2 and b being equal to 1 or 2.

10. Reagent according to any one of the preceding claims, characterised in that the said hydrophobic polymer is chosen from the group consisting of polystyrene, polyvinyltoluene, polypropylene, polyvinyl chloride, polyvinylidene chloride and their copolymers.

11. Reagent according to any one of the preceding claims, characterised in that the said hydrophobic polymer is polystyrene and that the said side chains are -CH₂-Z-CHZ'(Z'') and optionally -CH₂-Z-CHZ₁(Z₂) chains, Z, Z' and Z'' being defined as in Claim 1, and Z₁ and Z₂ being defined as in Claim 2.

12. Reagent according to any one of Claims 1, 2 and 6 to 11, characterised in that it contains side chain with a =YL or -NH-L end, Y being defined as in Claim 1 and L being the residue of an amino ligand chosen from proteins, in particular antibodies, antigens, haptens, peptide hormones, enzymes or oligonucleotides, lectins, neurotransmitters or natural or synthetic drugs agonistic or antagonistic for a neurotransmitter.

13. Reagent according to Claim 12, characterised in that the said antibody is a specific antibody recognizing antigens corresponding to pathogenic states.

14. Reagent according to Claim 13, characterised in that the said antibody is chosen from the group consisting of anti(fibrinogen degradation product) antibodies, antimeningococcus A antibodies and antimeningococcus B antibodies.

15. Reagent according to any one of the preceding Claims, characterised in that it is in the form of beads.

16. Reagent according to Claim 15, characterised in that it is in the form of microbeads whose dimensions may range approximately from 0.2 to 1 micrometre.

17. Reagent according to any one of the preceding claims, characterised in that the side chains of formula I are attached to the hydrophobic polymer by means of a group
-CH₂-CHX₁-(Ar)ₐ-CH₂-
where Ar is a m- or p-phenylene group optionally substituted by a methyl group, a is number equal to 0 or 1, and X₁ denotes H or CH₃.

18. Process for the preparation of a reagent such as defined in any one of the preceding claims, characterised in that the said solid support is prepared by polymerisation or copolymerisation of one or more hydrophobic unsaturated monomers, according to usual polymerisation methods, and that before the end of the polymerisation there is added to the reaction mixture a monomer with a hydrophilic chain, an end of which contains a double bond which is copolymerisable with the said hydrophobic unsaturated monomers and the other end of which contains an end group of formula -Z-CHZ₃(Z₄) in which Z (constituting the hydrophilic chain) is defined as in Claim 1, and Z₃ and Z₄ denote =O or a protective group for the carbonyl, the said hydrophilic monomer being optionally in a mixture with a similar hydrophilic monomer with a -Z-CH₂OH end group, that, in the case where Z₃ and Z₄ denote a protective group for the carbonyl, a deprotection reaction is carried out, that, if desired, the reagent obtained, which contains side chains of formula III:
-Z-CH=O (III)
is reacted with an amino ligand, according to known methods, to obtain a corresponding reagent in which a proportion of the side chains correspond to the formula IV:
-Z-CHY'L (IV)
in which Z is defined as above, Y' denotes an =N- group bonded to the CH group via its double bond, or Y' denotes an = 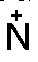 = group and L denotes the residue of the ligand molecule,
that, if desired, the reagent obtained is reacted with a primary amine of formula RNH₂, R being an optionally hydroxylated lower alkyl containing 1 to 4 carbon atoms, so as to convert the -Z-CH=O side chains which have not reacted with the ligand into corresponding chains of formula V:
-Z-CH=N-R (V),
and that, in the case where the reagent contains side chains of formula IV and optionally of formula V, the reagent obtained is subjected, if desired, to the action of a reducing agent capable of reducing the imine or iminium groups, to obtain a corresponding reagent containing side chains of formula VI:
-Z-CH₂-NH-L (VI)
and, if appropriate, side chains of formula VII:
-Z-CH₂-NH-R (VII).

19. Process according to Claim 18, characterised in that, to prepare a reagent in the form of microbeads, the polymerisation is carried out according to the emulsion polymerisation technique.

20. Process according to Claim 18 or 19, characterised in that a hydrophilic monomer is employed, of formula IX: 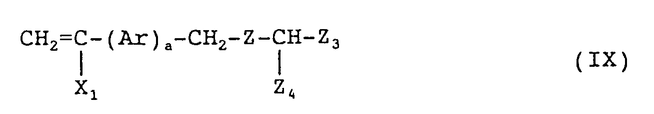 in which Z, Z₃ and Z₄ are defined as in Claim 18, and Ar, X₁ and a are defined as in Claim 17.

21. Use of a reagent according to any one of Claims 1 to 17, characterised in that
a) in the case where the reagent does not contain side chains of formula I with a =YL or -NH-L end the reagent containing side chains with an aldehyde end is brought into contact with a solution of an amino ligand to obtain a reagent containing side chains of formula IV:
-Z-CHY'L (IV)
in which Z and L are defined as in Claim 1, and Y' is defined as in Claim 18, and, if desired, the aldehyde groups remaining on the side chains which have not reacted with the ligand are neutralised by reaction with a primary amine R-NH₂, R being defined as in Claim 2, and that, if desired, the reagent obtained is subjected to the action of a reducing agent capable of reducing the imine or iminium groups to corresponding amine groups; and/or
b) with the aid of the reagent containing side chains with a =YL or -NH-L end, L, Y and L being defined as in Claim 1, a search is made, according to methods which are known per se, for the possible presence of the receptor or of a partner of the said ligand in a solution or in a biological fluid to be examined; or
c) when the ligand attached to the reagent is an enzyme, the said reagent is used as a catalyst in biological, chemical or biochemical reactions catalysed by the said enzyme.

## Patentansprüche

1. Reagens, dadurch gekennzeichnet, daß es hauptsächlich aus einem festen Träger aus einem hydrophoben Polymerisat besteht an dessen Oberfläche mittels kovalenter Bindungen hydrophile Seitenketten der Formel I gebunden sind: 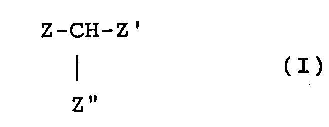 worin Z ein organischer Abstandshalterarm des hydrophilen, nicht hydroxylgruppenhaltigen Teils mit einer Kette aus 4 bis 70 Kohlenstoffatomen ist, Z' und Z'' zusammen =O, eine Carbonylschutzgruppe oder eine Gruppe =YL bedeutet, worin Y =N- oder =N⁺= darstellt und L den Rest eines Aminliganden der schematischen Formel L-NH₂ oder L=NH darstellt oder Z'' H bedeutet und Z' die Gruppe -NH-L bedeutet, worin L der Rest des genannten Liganden darstellt.

2. Reagens nach Anspruch 1, dadurch gekennzeichnet, daß es Seitenketten der Formel I mit der Endgruppe =YL oder -NH-L enthält und daß es weiterhin Seitenketten der Formel I enthält, wobei Z' und Z'' =O und/oder Seitenketten der Formel II darstellen 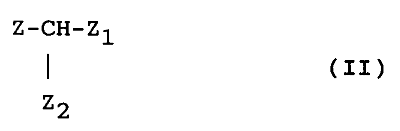 worin Z die Definition gemäß Anspruch 1 hat, Z₁ H und Z₂ -OH oder -NHR bedeuten oder Z₁ und Z₂ zusammen die Gruppe =N-R darstellen, worin R eine niedere Alkylgruppe ist, die gegebenenfalls hydroxyliert ist und 1 bis 4 Kohlenstoffatome besitzt.

3. Reagens nach den Ansprüchen 1 oder 2, dadurch gekennzeichnet, daß es Seitenketten der Formel I enthält, wobei Z'und Z'' zusammen =O darstellen, dar Rest der Seitenketten, die am Reagens vorhanden sind, Seitenketten der Formel II sind mit Z₁=H und Z₂=OH, und wobei die Ketten der Formel I mindestens 25 % der gesamten vorhandenen Seitenketten der Formel I darstellen, die am Reagens vorhanden sind.

4. Reagens nach Anspruch 1, dadurch gekennzeichnet, daß die am Reagens vorhandenen Seitenketten der Formel I solche sind, bei denen Z' und Z'' zusammen =O oder eine Carbonylschutzgruppe bedeuten.

5. Reagens nach Anspruch 4, dadurch gekennzeichnet, daß die Schutzgruppe eine Acetal-, Oxazin- oder Oxazolingruppe ist.

6. Reagens nach einem der Ansprüche 1 bis 5, dadurch gekennzeichnet, daß die Dichte der gesamten Seitenketten der Formel I, bezogen auf die Oberfläche des Reagens, etwa 10⁻⁵ bis 10⁻¹⁰ Mol/m² beträgt.

7. Reagens nach Anspruch 6, dadurch gekennzeichnet daß die Dichte etwa 10⁻⁵ bis 10⁻⁷ Mol/m² beträgt.

8. Reagens nach den vorhergehenden Ansprüchen, dadurch gekennzeichnet, daß z eine Polyoxyethylengruppe mit mindestens 2 Oxyethyleneinheiten ist.

9. Reagens nach Anspruch 8, dadurch gekennzeichnet, daß Z eine Gruppe -(OCH₂CH₂)ₙ₋₁OCH₂- oder - (OCH₂CH₂)ₙO-(CH₂)_{b}- darstellt, wobei n eine Zahl von mindestens 2 und b gleich 1 oder 2 ist.

10. Reagens nach den vorhergehenden Ansprüchen, dadurch gekennzeichnet, daß das hydrophobe Polymerisat ausgewählt ist aus der Gruppe bestehend aus Polystyrol, Polyvinyltoluol, Polypropylen, Polyvinylchlorid, Polyvinylidenchlorid und Copolymeren davon.

11. Reagens nach den vorhergehenden Ansprüchen, dadurch gekennzeichnet, daß das hydrophobe Polymerisat Polystyrol ist und daß die Seitenketten -CH₂-Z-CHZ'(Z'') und gegebenenfalls -CH₂-Z-CHZ₁(Z₂) sind, wobei Z, Z' und Z'' die in Anspruch 1 genannte Bedeutung haben und Z₁ und Z₂ wie in Anspruch 2 definiert sind.

12. Reagens nach den vorhergehenden Ansprüchen 1, 2 und 6 bis 11, dadurch gekennzeichnet, daß es Seitenketten mit der Endgruppe =YL oder -NH-L hat, wobei Y gemäß Anspruch 1 definiert ist und L der Rest eines Aminliganden ist, ausgewählt aus Protein, insbesondere Antikörpern, den Antigenen, den Haptenen, den Peptidhormonen, den Enzymen oder auch den Oligonucleotiden, den Lectinen, den Neurotransmittern oder den natürlichen oder synthetischen Arzneimitteln, die Agonisten oder Antagonisten der Neurotransmitter sind.

13. Reagens nach Anspruch 12, dadurch gekennzeichnet, daß der Antikörper ein Antikörper ist, der spezifisch das entsprechende Antigen im pathogenen Zustand erkennt.

14. Reagens nach Anspruch 13, dadurch gekennzeichnet, daß der Antikörper ausgewählt ist aus der Gruppe bestehend aus den Antikörpern, die Antiprodukte des Fibrinogen-Abbaus sind, den Anti-Meningokokken-A-Antikörpern und den Anti-Meningokokken-B-Antikörpern.

15. Reagens nach den vorherigen Ansprüchen, dadurch gekennzeichnet, daß es in Form von Kugeln vorliegt.

16. Reagens nach Anspruch 15, dadurch gekennzeichnet, daß es in Form von Mikrokugeln, die Dimensionen von etwa 0,2 bis 1 Mikrometer haben können, vorliegt.

17. Reagens nach den vorhergehenden Ansprüchen, dadurch gekennzeichnet, daß die Seitenketten der Formel I an das hydrophobe Polymerisat durch eine Zwischengruppe -CH₂-CHX₁-(Ar)ₐ-CH₂- gebunden sind, wobei Ar eine m- oder p-Phenylengruppe ist, die gegebenenfalls durch eine Methylgruppe substituiert ist und a eine Zahl 0 oder 1 ist und X₁ H oder CH₃ bedeutet.

18. Verfahren zur Herstellung eines Reagens gemäß den vorhergehenden Ansprüchen, dadurch gekennzeichnet, daß man den festen Träger durch Polymerisation oder Copolymerisation von einem oder mehreren ungesättigten hydrophoben Monomeren nach üblichen Polymerisationsmethoden herstellt und vor dem Polymerisationsende im reaktiven Medium ein Monomer zugibt, das eine hydrophile Kette besitzt und an einem Ende eine mit den ungesättigten hydrophoben Monomeren copolymerisierbare Doppelbindung aufweist und am anderen Ende eine Endgruppe der Formel -Z-CHZ₃(Z₄) besitzt, wobei Z (bestehend aus der hydrophilen Kette) gemäß Anspruch 1 definiert ist und Z₃ und Z₄ =O oder eine Carbonylschutzgrupe darstellen, wobei das hydrophile Monomere gegebenenfalls im Gemisch mit einem hydrophilen Monomeren vorliegt, das eine analoge Endgruppe -Z-CH₂OH besitzt, welche im Fall, daß Z₃ und Z₄ eine Carbonylschutzgruppe darstellen, eine Schutzreaktion bewirken, welche, falls gewünscht, mit dem erhaltenen Reagens reagieren kann, welches Seitenketten der Formel III trägt
-Z-CH=O (III)
mit einem Aminliganden, nach bekannten Methoden, um ein Reagens entsprechend dem Teil der Seitenketten zu erhalten, welche der Formel IV entsprechen:
-Z-CHY'L (IV)
worin Z wie vorher definiert ist, Y' eine Gruppe =N- ist, die durch ihre Doppelbindung mit einer Gruppe CH gebunden ist, oder Y' eine Gruppe =N⁺= ist und L den Rest des Ligandenmoleküls bedeutet, und daß man, falls gewünscht, das erhaltene Reagens mit einem primären Amin der Formel RNH₂ umsetzt, worin R eine niedere, gegebenenfalls hydroxylierte Alkylgruppe mit 1 bis 4 Kohlenstoffatomen ist, derart, daß die Seitenketten -Z-CH=O, die nicht mit dem Liganden reagiert haben, in entsprechenden Ketten der Formel V überführt werden:
Z-CH=N-R (V)
und daß in dem Fall, daß das Reagens Seitenketten der Formel IV und gegebenenfalls der Formel V hat, man das erhaltene Reagens, falls gewünscht, der Einwirkung eines Reduktionsmittels unterwirft, das in der Lage ist, die Imin- oder Imminiumgruppen zu reduzieren zwecks Erzielung eines entsprechenden Reagens mit Seitenketten der Formel VI
-Z-CH₂-NH-L (VI)
und gegebenenfalls Seitenketten der Formel VII
-Z-CH₂-NH-R (VII)

19. Verfahren nach Anspruch 18, dadurch gekennzeichnet, daß man zur Herstellung eines Reagens unter Bildung von Mikrokugeln die Polymerisation gemäß einer Emulsions-Polymerisationstechnik durchführt.

20. Verfahren nach Anspruch 18 oder 19, dadurch gekennzeichnet, dass man ein hydrophiles Monomeres der formel IX 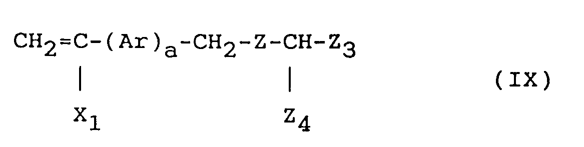 verwendet, worin Z, Z₃ und Z₄ die in Anspruch 18 genannte Bedeutung haben und Ar, X₁ und a gemäß Anspruch 17 definiert sind.

21. Verwendung eines Reagens nach den Ansprüchen 1 bis 17, dadurch gekennzeichnet, daß man
a) im Fall, daß es keine Seitenketten der Formel I mit der Endgruppe =YL oder -NH-L besitzt, das Reagens mit Seitenketten mit Aldehyd-Endgruppen in Kontakt mit einer Lösung eines Aminliganden bringt, um ein Reagens zu erhalten, das Seitenketten der Formel IV hat:
-Z-CHY'L (IV)
worin Z und L gemäß Anspruch 1 definiert sind und Y' gemäß Anspruch 18 definiert ist, und daß man, falls gewünscht, die restlichen Aldehydruppen an Seitenketten, die nicht mit dem Liganden reagiert haben, durch Reaktion mit einem primären Amin R-NH₂, worin R gemäß Anspruch 2 definiert ist, umsetzt und daß man, falls gewünscht, das erhaltene Reagens der Wirkung eines Reduktionsmittels aussetzt, das in der Lage ist, die Imin- oder Imminiumgruppen zu entsprechenden Amingruppen zu reduzieren; und/oder
b) mittels des Reagens, das Seitenketten mit Endgruppen =YL oder NH-L hat, wobei L und Y gemäß Anspruch 1 definiert sind, man nach an sich bekannten Verfahren die eventuelle Gegenwart eines Rezeptors oder eines Teils des Liganden in Lösung oder in einer flüssigen biologischen Prüfsubstanz prüft; oder
c) wenn der am Reagens fixierte Ligand ein Enzym ist, man das Reagens als Katalysator für biologische, chemische oder biochemische Reaktionen benützt, die durch das Enzym katalysiert werden.
